# EUROPEAN PATENT APPLICATION

(11) **EP 1 842 498 A1**
(43) Date of publication of application: **10.10.2007**
(21) Application number: 05822453.6
(22) Date of filing: 29.12.2005
(51) Int. Cl.: A61B 17/28, A61B 1/00, A61B 17/32

(54) **TREATMENT DEVICE FOR ENDOSCOPE**

(30) Priority: 06.01.2005 JP 2005001359
(71) Applicant: Olympus Corporation, Tokyo 151-0072 (JP)
(72) Inventor: SUZUKI, Keita, 1900013 (JP)
(74) Representative: von Hellfeld, Axel
(86) International application number: PCT/JP2005/024153
(87) International publication number: WO 2006/073128

(57) **Abstract**

A treatment tool (1) for an endoscope includes: an elongate-flexible tube (5); a second forceps piece (8) which is disposed at the tip of the flexible tube (5); a first forceps piece (7) which is disposed at the tip of the flexible tube (5) and which moves so as to open from or close against the second forceps piece (8); and a guide portion (12) which is provided in the second forceps piece (8) so as to protrude forward of the tip of the first forceps piece (7) along with extending in the longitudinal direction of the second forceps piece(8).

## Description

### TECHNICAL FIELD

The present invention relates to a treatment tool for an endoscope.
The present application claims the priority of Japanese Patent Application No. 2005-001359 filed on January 6, 2005, and incorporates the contents thereof herein by reference.

### BACKGROUND ART OF THE INVENTION

Recently, there have been many cases of performing an endoscopic treatment for diseases in the alimentary duct system or pancreaticobiliary duct system. In the current treatment for the pancreaticobiliary duct system using an endoscope, a therapeutic treatment which includes recovering gallstones exsisting in the biliary duct using a balloon and a grasping device is performed, along with a diagnostic treatment which includes angiography of the pancreaticobiliary duct.

With the endoscopic treatment for such as the pancreatic duct, the biliary duct and the hepatic duct, an incision of the duodenal papilla using a papillotomy knife is performed. For the incision, first, the tip of an insertion portion of the endoscope is inserted into the vicinity of the duodenal papilla. Then, the papillotomy knife is selectively inserted into the pancreatic duct or the biliary duct while guiding the papillotomy knife along a guide wire which has been placed in the body under X-ray illumination (for example, refer to Patent documents 1 and 2 as recited below).
Further, in a case where a constriction of a tumor is formed in the biliary duct or the like, in order to secure a lumen through which bile or pancreatic fluid can passed, the tube stent is inserted into the biliary duct or the like. Then, the tube stent is retracted from the biliary duct or the like as required.

However, in the case where the treatment using the papillotomy knife is performed as described above, an incising operation such as an adjustment of the length of the incision is complicated.
Further, if a high-frequency forceps which can be used to incise an object only when two forceps pieces which form the high-frequency forceps are closed against each other (for example, refer to Patent document 1 as recited below) is used instead of the papillotomy knife, since it is hard to insert the high-frequency knife into the biliary duct or the like, the duodenal papilla can be incised while grasping the duodenal papilla stably.
Furthermore, when a tube stent is removed from the biliary duct, the treatment using the tool such as a snare takes a long time, thereby it is hard to retract the tube from the biliary duct.
PATENT DOCUMENT 1: Japanese Unexamined Patent Application, First Publication No. H11-033033
PATENT DOCUMENT 2: Japanese Unexamined Patent Application, First Publication No. H11-128240
PATENT DOCUMENT 3: Japanese Unexamined Patent Application, First Publication No. H05-253241

### DETAILED DESCRIPTION OF THE INVENTION

### PROBLEMS TO BE SOLVED BY THE INVENTION

The present invention has been developed in view of the above-described situations, and it is therefore an object of the invention to provide a treatment tool for an endoscope which enables the object to be easily grasped or incised in a condition in which the object is stable, and to perform the operation in a short time.

### MEANS FOR SOLVING THE PROBLEMS

A treatment tool for an endoscope of the present invention includes: an elongate-flexible tube; a first forceps piece and a second forceps piece which are disposed at a tip of the flexible tube and which move so as to be separate and close each other; and a guide portion which is provided to the second forceps piece so as to protrude forward of the tip of the first forceps piece along with extending in a longitudinal direction of the second forceps piece.

According to the treatment tool for an endoscope of the present invention, for example, in a case where a tube-shaped treated object is grasped or incised in a longitudinal direction of the treated object, it is possible to stably attach the treated object to the second forceps piece by inserting the guide portion to the inside of the treated object. Then, it is possible to grasp or incise the treated object easily by the first forceps piece and the second forceps piece being moved so as to be open and close while maintaining the condition.

A treatment tool for an endoscope of the present invention includes: an elongate-flexible tube; a first forceps piece and a second forceps piece which are disposed at a tip of the flexible tube; and a flexible guide portion which is provided to the second forceps piece so as to protrude forward of the tip of the first forceps piece along with extending in a longitudinal direction of the second forceps piece. The first forceps piece moves so as to open and close with respect to the second forceps piece.

According to the treatment tool for an endoscope of the present invention, for example, in a case where a tube-shaped treated object is grasped or incised in a longitudinal direction of the treated object, it is possible to stably attach the treated object to the second forceps piece by inserting the flexible guide portion to the inside of the treated object. Then, it is possible to grasp or incise the treated object by the first forceps piece being moved so as to open and close with respect to the second forceps piece while maintaining the condition. At this time, since the second forceps piece which is provided with the guide portion is not moved, it is possible to grasp or incise the treated object stably.

In the treatment tool for an endoscope of the present invention, it may be arranged such that the guide portion be formed so as to be integrated with the second forceps piece.

According to the treatment tool for an endoscope of the present invention, just only the first forceps piece is fitted to the second forceps piece while assembling the treatment tool, and the predetermined-length guide portion protrudes forward of the tip of the flexible tube. Therefore, it is possible to assemble the treatment tool easily.

In the treatment tool for an endoscope of the present invention, it may be arranged such that the guide portion be provided with a lumen which is formed in a longitudinal direction of the guide portion and which opens at the tip of the guide portion.
According to the treatment tool for an endoscope of the present invention, tools which are needed for treatment can be inserted into the lumen. Therefore, it is possible to move the tools through the lumen. Further, it is possible to move contents forward of the tip of the guide portion through the lumen.

In the treatment tool for an endoscope of the present invention, it may be arranged such that the lumen be formed so that a different treatment tool for an endoscope is insertable into the lumen.
In the treatment tool for an endoscope, the lumen can be used as if it is the treatment tool insertion channel which is provided at the endoscope.

A treatment tool for an endoscope of the present invention includes: an elongate-flexible tube which can be inserted into a treatment tool insertion channel provided at an endoscope being insertable into a body cavity and which protrudes from the tip of the endoscope; a second forceps piece which is disposed at the tip of the flexible tube; a first forceps piece which is disposed at the tip of the flexible tube and which grasps a treated object with the second forceps piece by moving so as to open and close with respect to the second forceps piece; and a flexible guide portion which is provided at the second forceps piece so as to protrude forward of the tip of the first forceps piece along with extending in a longitudinal direction of the second forceps piece and which extends to a proximal section of the endoscope along the flexible tube.

In the treatment tool for an endoscope, for example, in a case where a tube-shaped treated object is grasped or incised in a longitudinal direction of the treated object, it is possible to stably attach the treated object to the second forceps piece by inserting the guide portion to the inside of the treated object. Then, it is possible to grasp or incise the treated object by the first forceps piece being moved so as to open and close with respect to the second forceps piece while maintaining the condition. At this time, since the second forceps piece which is provided with the guide portion is not moved, it is possible to grasp or incise the treated object stably. Further, it is possible to operate the guide portion at the proximal section of the endoscope separately from the operation of the forceps pieces.

### ADVANTAGEOUS EFFECTS OF THE INVENTION

According to the present invention, it is possible to perform the procedure easily, and it is possible to perform the treatment using the endoscope within a short time.

### BRIEF DESCRIPTIONS OF THE DRAWINGS

[FIG. 1] FIG. 1 is a schematic view showing a treatment procedure using an endoscopic forceps of a first embodiment of the present invention.
[FIG. 2] FIG. 2 is a side view, partly broken away, showing a significant part of the endoscopic forceps of the first embodiment of the present invention.
[FIG. 3] FIG. 3 is a schematic view showing a modification of the endoscopic forceps of the first embodiment of the present invention, and shows the treatment procedure using the endoscopic forceps.
[FIG. 4] FIG. 4 is a side view, partly broken away, showing another modification of the endoscopic forceps of the first embodiment of the present invention, and shows a significant part of the endoscopic forceps.
[FIG. 5] FIG. 5 is a schematic view showing a treatment procedure using a high-frequency incision forceps of a second embodiment of the present invention.
[FIG. 6] FIG. 6 is a sectional view showing the high-frequency incision forceps of the second embodiment of the present invention.
[FIG. 7] FIG. 7 is a side view showing the high-frequency incision forceps of the second embodiment of the present invention as seen in a direction of an arrow B in FIG. 9.
[FIG. 8] FIG. 8 is a side view, partly broken away, showing a significant part of the high-frequency incision forceps of the second embodiment of the present invention.
[FIG. 9] FIG. 9 is a side view, partly broken away, showing the significant part of the high-frequency incision forceps of the second embodiment of the present invention as seen in a different direction to FIG. 8.
[FIG. 10] FIG. 10 is a side view showing the significant part of the high-frequency incision forceps of the second embodiment of the present invention as seen in a direction of an arrow B in FIG. 9.
[FIG. 11] FIG. 11 is a side view showing a significant part of the high-frequency incision forceps of a third embodiment of the present invention.
[FIG. 12] FIG. 12 is a side view, partly broken away, showing the significant part of the high-frequency incision forceps of the third embodiment of the present invention.
[FIG. 13] FIG. 13 is a side view showing a significant part of the high-frequency incision forceps of a fourth embodiment of the present invention.
[FIG. 14] FIG. 14 is a side view, partly broken away, showing the significant part of the high-frequency incision forceps of the fourth embodiment of the present invention.
[FIG. 15] FIG. 15 is a side view showing a significant part of the high-frequency incision forceps of a fifth embodiment of the present invention.
[FIG. 16] FIG. 16 is a side view, partly broken away, showing a significant part of the high-frequency incision forceps of a sixth embodiment of the present invention.
[FIG. 17] FIG. 17 is a side view showing a significant part of the high-frequency incision forceps of a seventh embodiment of the present invention.
[FIG. 18] FIG. 18 is a sectional view taken along a line C-C in FIG. 17 showing the significant part of the high-frequency incision forceps of the seventh embodiment of the present invention.

### DESCRIPTION OF THE REFERENCE SYMBOLS

- 1 and 19: ENDOSCOPIC FORCEPS (TREATMENT TOOL FOR ENDOSCOPE)
- 5: FLEXIBLE TUBE
- 7 and 21: FIRST FORCEPS PIECE
- 8 and 22: SECOND FORCEPS PIECE
- 12, 25, 36, 46, 48, 51 and 55: GUIDE PORTION
- 16: ENDOSCOPE
- 17A: TREATMENT TOOL INSERTION CHANNEL
- 20, 35, 45, 50, 53 and 57: HIGH-FREQUENCY INCISION FORCEPS (TREATMENT TOOL FOR ENDOSCOPE)
- 26 and 56: TREATMENT SURFACE
- 37 and 47: LUMEN

### DESCRIPTION OF THE PREFERRED EMBODIMENTS

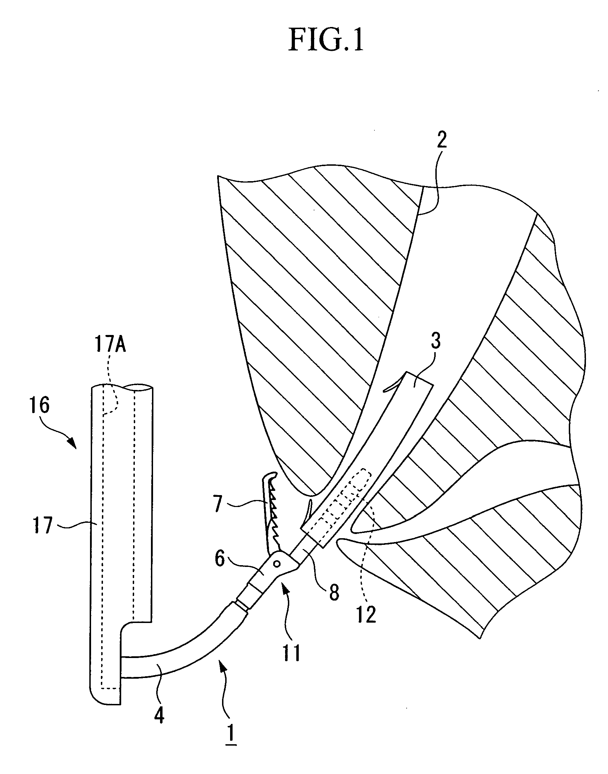

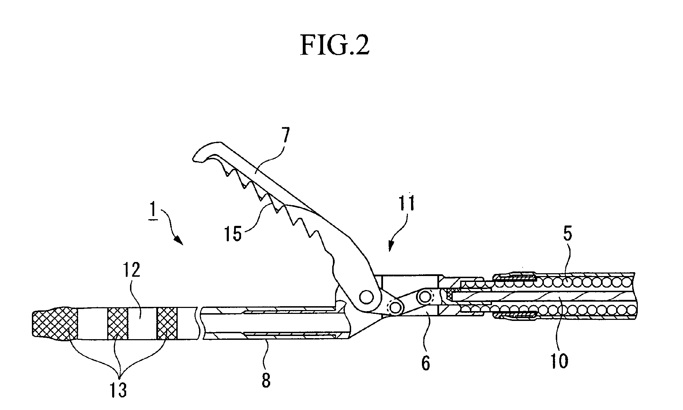

A first embodiment of the present invention will be explained with reference to FIG. 1 and FIG. 2.
An endoscopic forceps (that is, a treatment tool for an endoscope) 1 of this embodiment is used for removing from a tubular stent 3 placed in the biliary duct 2. The forceps 1 includes an elongated flexible tube 5, a first forceps piece 7, a second forceps piece 8, an operation wire 10, a link mechanism 11 and an operation section (not shown).

The flexible tube 5 is formed like a coil, and is covered with an insulation sheath 4. The first forceps piece 7 and the second forceps piece 8 are attached to the tip of the flexible tube 5 through a tip cover 6. The tip of the operation wire 10 is connected to the first forceps piece 7. The link mechanism 11 converts a forward and backward movement of the operation wire 10 into an opening and closing movement of the first forceps piece 7 and the second forceps piece 8. The operation section is operated by an operator, and moves the operation wire 10 forward and backward with respect to the flexible tube 5.

A guide portion 12 is formed so as to be integrated with the second forceps piece 8. The guide portion 12 is provided so as to protrude forward of the tip of the first forceps piece 7 along with extending in the longitudinal direction of the second forceps piece 8.
On the surface of the guide portion 12, markings 13 to indicate the insertion distance of the guide portion 12 are disposed so as to indicate the location at regular intervals. The markings 13 are for showing the insertion distance of the guide portion 12 into a treated object.
On a side face of the first forceps piece 7 facing the second forceps piece 8, a plurality of projections 15 are formed so as to easily grasp a living body tissue as the treated object, the tubular stent 3 or the like. Similarly, a plurality of projections 15 are formed on a side face of the second forceps piece 8 (including the guide portion 12) facing the first forceps piece 7.

The endoscopic forceps 1 is insertable into a treatment tool insertion channel 17A which is provided at an insertion portion 17 of an endoscope 16. In this embodiment, since the treatment for the biliary duct 2 through the duodenal papilla 18 is exemplified, a lateral vision type endoscope is used as the endoscope 16.

Next, the method to use the endoscopic forceps 1 of the present embodiment while the tube stent is retracted using the endoscopic forceps 1 will be described below. In addition, functions and effects of the endoscopic forceps 1 will be also described below.
First, the insertion portion 17 of the endoscope 16 is inserted into the body cavity, and then the tip of the insertion portion 17 is moved near the duodenal papilla 18. Next, the endoscopic forceps 1 is inserted into the treatment tool insertion channel 17A of the insertion portion 17, and then the first forceps piece 7 and the second forceps piece 8 protrude from the tip of the insertion portion 17.

Next, the operation wire 10 is moved forward of the tip of the flexible tube 5 by operating the unshown operation section, and thereby the link mechanism 11 is driven. As a result, the forceps piece 7 separates from the second forceps piece 8. That is, the first forceps piece 7 and the second forceps piece 8 open against each other.
While maintaining the condition where the forceps piece 7 separates from the second forceps piece 8, as shown in FIG. 1, the guide portion 12 is inserted to the inside of the tubular stent 3, and then the second forceps piece 8 is stably engaged with the tubular stent 3.

Next, the operation wire 10 is moved backward of the proximal end of the flexible tube 5 by operating the unshown operation section, and thereby the link mechanism 11 is driven so that the forceps piece 7 approaches the second forceps piece 8, and then the first forceps piece 7 and the second forceps piece 8 close against each other. As a result, the projections 15 bite into a wall of the tubular stent 3, and thereby the tubular stent 3 is grasped by the first forceps piece 7 and the second forceps piece 8.
Next, as a result of the insertion portion 17 of the endoscope 16 being retracted from the body cavity while grasping the tubular stent 3 by the first forceps piece 7 and the second forceps piece 8, the tubular stent 3 is removed from the body.

According to this endoscopic forceps 1, it is possible to stably put the tubular stent 3 on the second forceps piece 8 by inserting the guide portion 12 to the inside of the tubular stent 3. Further, it is possible to easily grasp the tubular stent 3 by closing the first forceps piece 7 and the second forceps piece 8.

According to this endoscopic forceps 1, since the guide portion 12 is formed so as to be integrated with the second forceps piece 8, only the first forceps piece 7 is fitted to the second forceps piece 8 while assembling of the forceps, and thereby the predetermined-length guide portion 12 protrudes forward of the tip of the flexible tube 12. Therefore, it is possible to assemble the forceps easily.

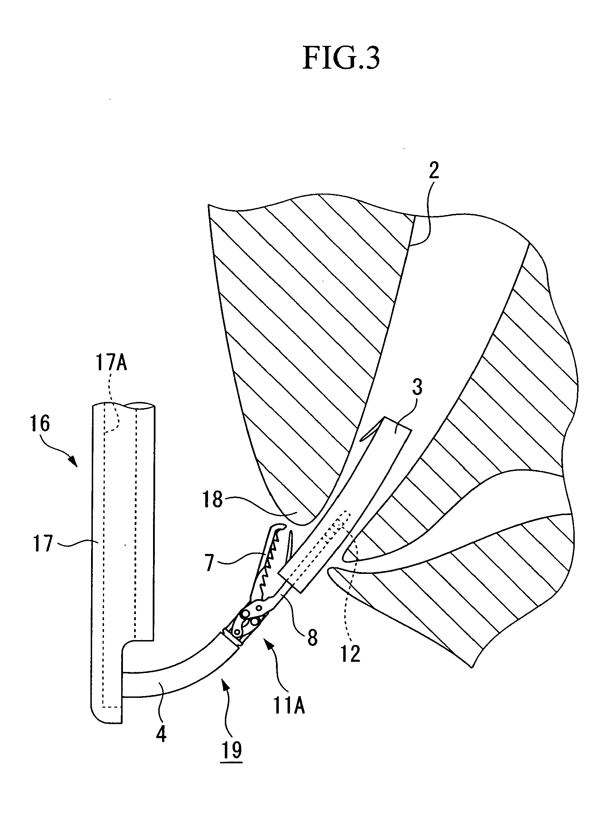

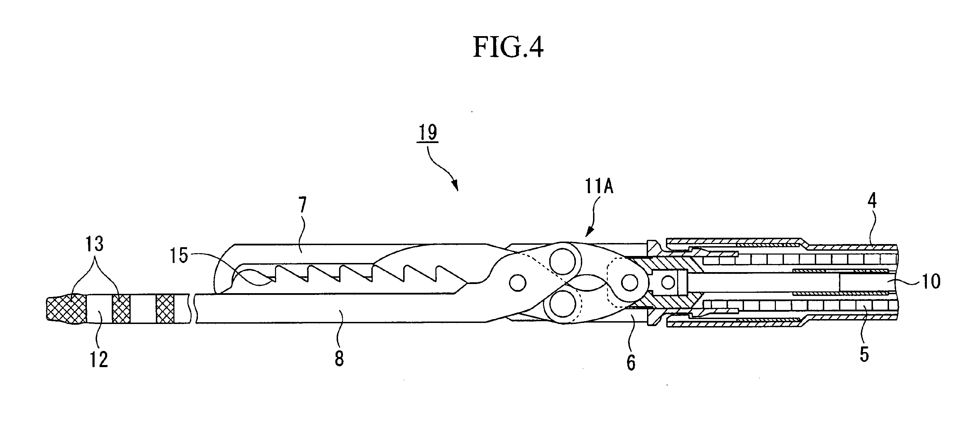

A modification of the endoscopic forceps of this embodiment is shown in FIG. 3 and FIG 4. An endoscopic forceps 19 includes a link mechanism 11A so that the first forceps piece 7 and the second forceps piece 8 move separately and closely to each other. According to this endoscopic forceps 19 a so-called "double swingable action" can produce the same effects as the endoscopic forceps 1 of the above-mentioned first embodiment's so-called "single swingable action".

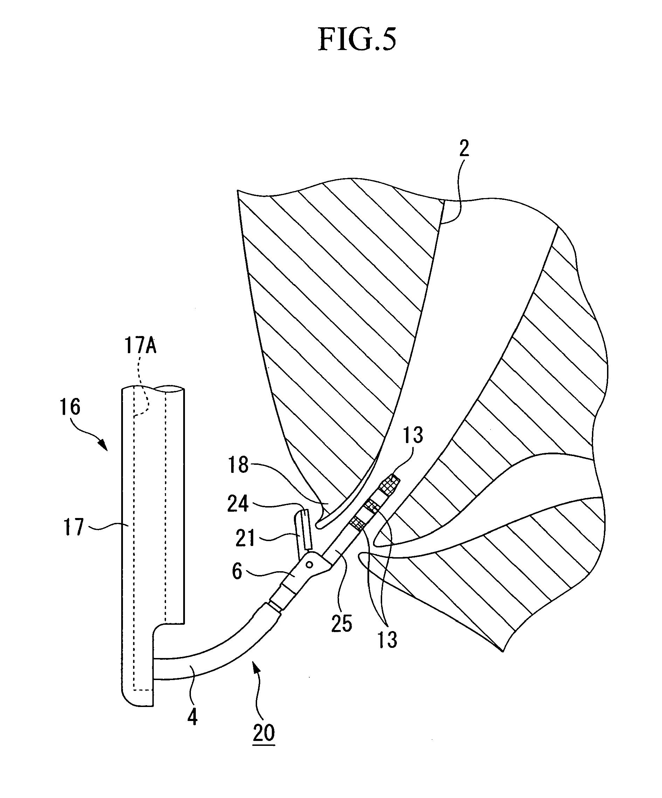

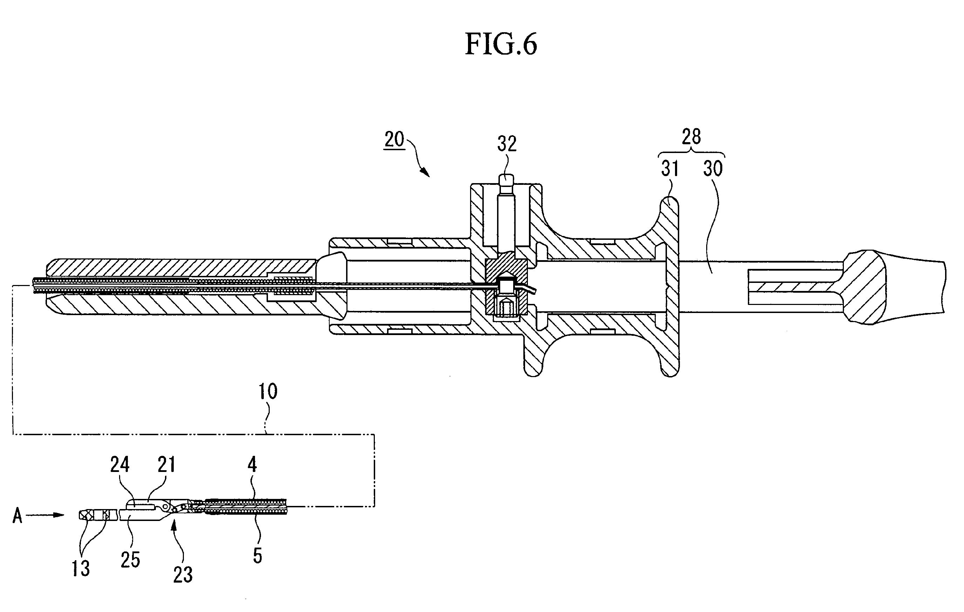

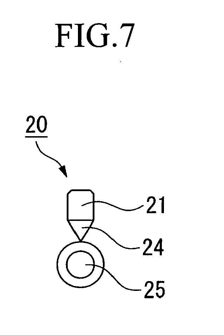

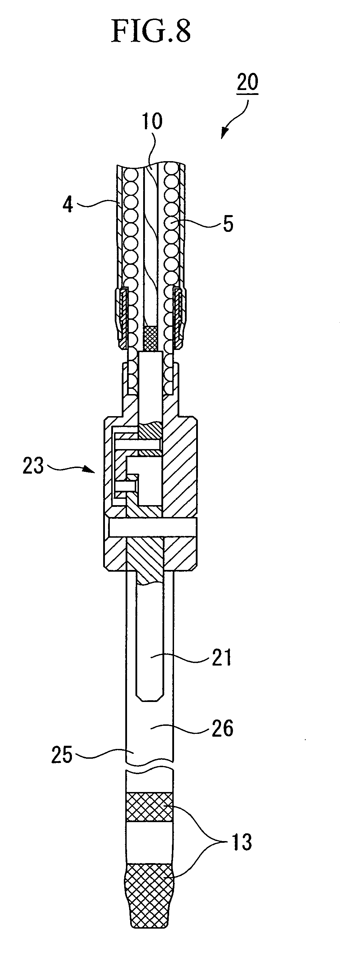

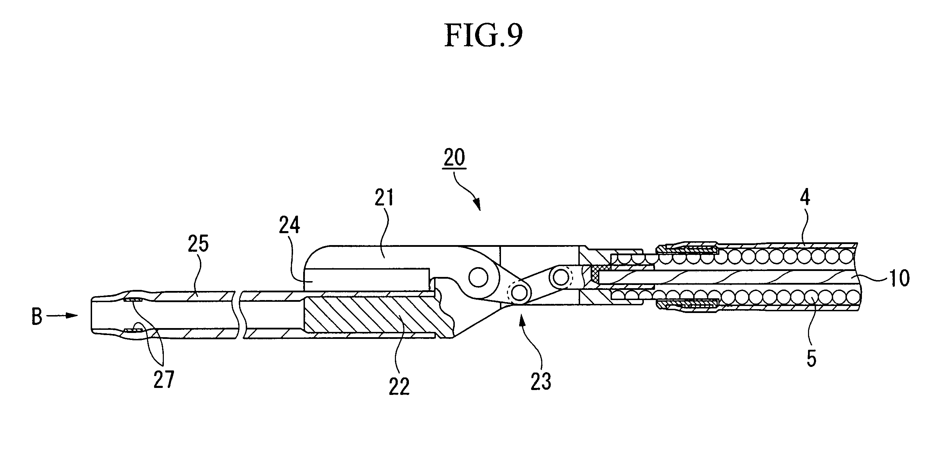

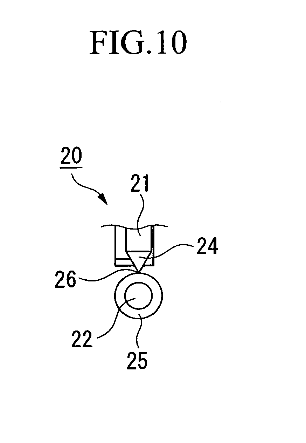

Next, a second embodiment of the present invention will be explained with reference to FIG. 5 through FIG. 10. Note that the same components included in the first embodiment are given the same reference numerals, and a description thereof is omitted.
In this embodiment, for example, as shown in FIG. 5, a high-frequency incision forceps (that is, a treatment tool for an endoscope) 20 is used for duodenal papilla surgery. As shown in FIG. 6 through FIG. 10, a first forceps piece 21 of the high-frequency incision forceps 20 is disposed at the tip of the flexible tube 5. The base end of the first forceps piece 21 is provided at the flexible tube 5 so as to be swingable with respect to the flexible tube 5. The first forceps piece 21 can be closed to and separated from a second forceps piece 22 through a link mechanism 23.
On the first forceps piece 21, the projections 15 mentioned in the first embodiment are not formed. In stead of the projections 15, an electrode 24 is disposed on a side face of the first forceps piece 21 facing the second forceps piece 22.
For increasing the current density, an edge of the electrode 24 is sharp so as to be tapered as close to the second forceps piece 22. A part of the first forceps piece 21 which protrudes from the tip cover 6 is coated with an insulation material such as ceramic (alumina, zirconia or the like) or fluorine-containing resin (PTEE, ETEE or the like). Therefore, when the first forceps piece 21 grasps the treated object with the second forceps piece 22, the electrode 24 linearly contacts the treated object.

The second forceps piece 22 is formed like a solid cylinder, and a flexible guide portion 25 having the electrical insulation property is provided at the tip of the second forceps piece 22 so as to protrude forward of the first forceps piece 21. The guide portion 25 is engaged with a part of the second forceps piece 22 facing to the first forceps piece 21. The part is covered by the guide portion 25, and the surface of the guide portion is formed as a treatment surface 26 which grasps the living body tissue with the first forceps piece 21.
On the outer surface of the guide portion 25, markings 13 as in the first embodiment are disposed. The markings 13 are for showing the insertion distance of the guide portion 12 into a treated object. Further, on the inner surface of the guide portion 25 which is close to the tip of the guide portion 25, an X-Ray marker 27 is disposed.

To the proximal end of the flexible tube 5, an operation section 28 is connected. The operation section 28 includes a rod-shaped operation section body 30 and a sliding portion 31. The operation section body 30 extends so as to be parallel with the proximal end of the operation wire 10. The sliding portion 31 can be moved back and forth along the longitudinal direction of the operation main body 30. The distal end of the operation wire 10 is connected to the sliding portion 31. Further, the sliding portion is provided with an electrical contact 32 which is connected to a high-frequency power source (not shown).

Next, the method to use the high-frequency incision forceps 20 of the present embodiment when the incision of a duodenal papilla is performed will be described below. In addition, effects of the high-frequency incision forceps 20 will also be described below.
First, similarly to the first embodiment, the insertion portion 17 of the endoscope 16 is inserted into the body cavity, and then the tip of the insertion portion 17 is moved to the vicinity of the duodenal papilla 18. Next, the high-frequency incision forceps 20 is inserted into the treatment tool insertion channel 17A of the insertion portion 17, and then the first forceps piece 21 and the second forceps piece 22 protrude from the tip of the insertion portion 17.

Next, the operation wire 10 is moved forward of the distal end of the flexible tube 5 by moving forward the sliding portion 31 with respect to the operation section body 30, and thereby the link mechanism 23 is driven. As a result, the forceps piece 21 separates from the second forceps piece 22. That is, the first forceps piece 21 and the second forceps piece 22 open from each other.
While maintaining the condition where the forceps piece 21 separates from the second forceps piece 22, as shown in FIG. 5, the guide portion 25 is inserted to the inside of the biliary duct 2. At this time, the X-Ray marker 27 can be visible by being illuminated with X-Ray. Therefore, it is possible to acknowledge the position of the guide portion 25.

Next, the operation wire 10 is moved backward of the proximal end of the flexible tube 5 by moving backward the sliding portion 31 with respect to the operation section body 30, and thereby the link mechanism 23 is driven so that the forceps piece 21 approaches the second forceps piece 21, and then the first forceps piece 7 and the second forceps piece 8 close against each other. As a result, a predetermined part which need to be incised such as the duodenal papilla 18 is grasped by the first forceps piece 21 and the second forceps piece 22.

Next, while grasping the predetermined part to be incised by the first forceps piece 21 and the second forceps piece 22, the electrode 24 is applied with high-frequency current through an electrical contact 32 and the operation wire 10 by operating an unshown high-frequency power source. As a result of applying the current to the electrode 24, a part which is grasped by the first forceps piece 21 and the second forceps piece 22 is cauterized, and thereby the part is incised along the edge of the electrode 24. After the living body tissue which is grasped by the first forceps piece 21 and the second forceps piece 22 has been incised, current to the electrode 24 is stopped by operating the high-frequency power source. Then, similarly to the first embodiment, the insertion portion 17 of the endoscope 16 is retracted from the body cavity.

According to the high-frequency incision knife 20, the first forceps piece 21 can be operated in a condition in which the second forceps piece 22 is substantially held in place by inserting the guide portion 25 into the biliary duct 2.
Therefore, compared with the papillotomy knife that is a conventionally arched high-frequency knife, the predetermined part to be incised can be grasped in a condition such that the guide portion 25 is inserted into the biliary duct 2. As a result, the living body tissue can be incised stably.
In addition, since the guide portion 25 has the electrical insulation property, and the electrode 24 disposed on the first forceps piece 21 does not contact the second forceps piece 22, the insulation can be reliably ensured.

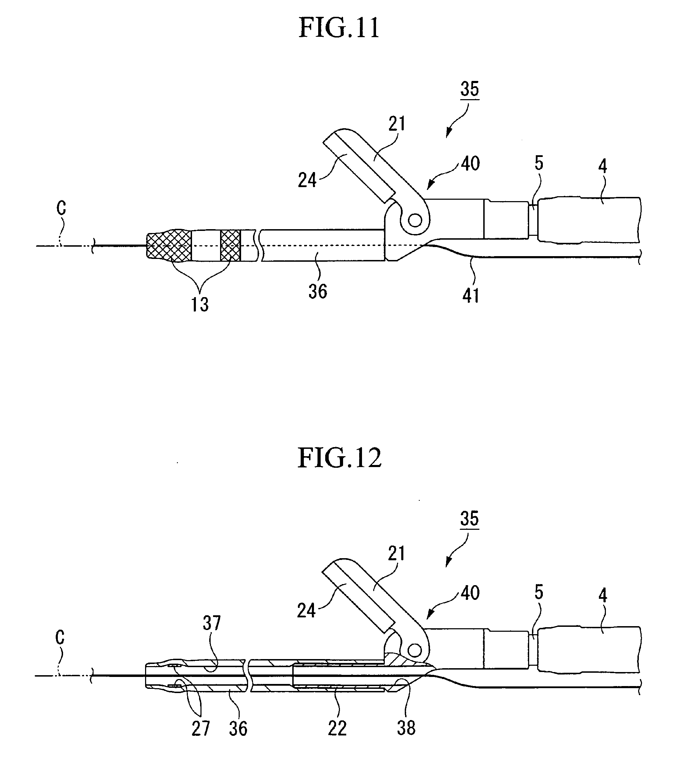

Next, a third embodiment of the present invention will be explained with reference to FIG. 11 and FIG. 12. Note that the same components included in the first and second embodiments are given the same reference numerals, and a description thereof is omitted.
A high-frequency incision forceps (that is, a treatment tool for an endoscope) 35 of the present invention is provided with a guide portion 36 having a lumen 37. The lumen 37 is formed so as to be along the longitudinal direction of the guide portion 36, and an aperture of the lumen 37 is formed at the tip of the guide portion 36. In addition, a through-hole 38 which communicates with the lumen 37 is formed on the second forceps piece 22. The through-hole 38 and the lumen 37 are formed so as to run along a straight line.

A link mechanism 40 closes the first forceps piece 21 against the second forceps piece 22 or opens the first forceps piece 21 from the second forceps piece 22.
The link mechanism 40 is located closer to the first forceps piece 21 than a center axis C of the lumen 37 so that the guide wire 41 does not interfere with the link mechanism 40 when the guide wire 41 is inserted into the lumen 37.

Next, the method to use the high-frequency incision forceps 35 of the present embodiment when the incision of a duodenal papilla is performed will be described below. In addition, that is, effects of the high-frequency incision forceps 35 will also be described below.
First, similarly to the first and second embodiments, the insertion portion 17 of the endoscope 16 is inserted into the body cavity, and then the tip of the insertion portion 17 is moved to the vicinity of the duodenal papilla 18. Next, the guide wire 41 is inserted into the biliary duct through a treatment tool insertion channel (not shown) and is placed at a desired position in the biliary duct in accordance with a common method and operation, and then the guide wire 41 is inserted into the lumen 37 and the through-hole 38 from the tip of the guide portion 36.

Next, the high-frequency incision forceps 35 is inserted into the treatment tool insertion channel (not shown) while the high-frequency incision forceps 35 is guided along the guide wire 41, and then the first forceps piece 21 and the second forceps piece 22 protrude from the tip of the endoscope.
Next, the second forceps piece 22 is inserted into the biliary tract while the second forceps piece 22 is guided along the guide wire 41. When the first forceps piece 21 and the second forceps piece 22 have moved to the predetermined position to be incised, similar to the second embodiment, the predetermined treatment is performed by operating the first forceps piece 21 and the second forceps piece 22.

According to the high-frequency incision forceps 35, the guide wire 41 is placed inside the biliary tract in advance, and then the guide wire 41 is inserted into the lumen 37. Thereby, the high-frequency incision forceps 35 is inserted into the biliary tract and the high-frequency incision forceps 35 is guided to the tip of the endoscope. As a result, the high-frequency incision forceps 35 can be moved easily.
In addition, since the link mechanism 41 does not interfere with the guide wire 41, the guide wire 41 can be operated smoothly.

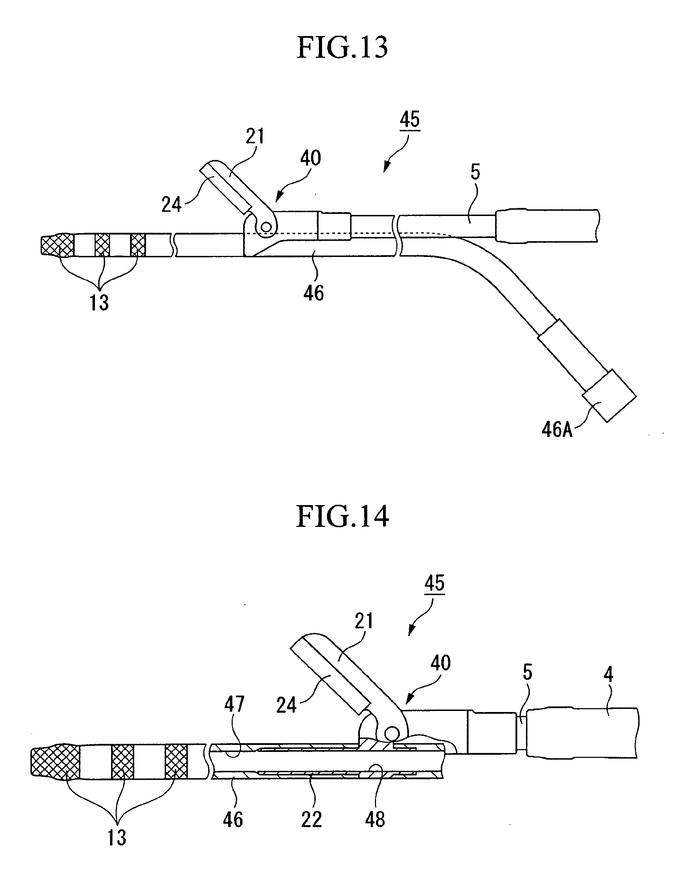

Next, a fourth embodiment of the present invention will be explained with reference to FIG. 13 and FIG. 14. Note that the same components included in the first, second or third embodiments are given the same reference numerals, a description thereof is omitted.
A high-frequency incision forceps (that is, a treatment tool for an endoscope) 45 of the present invention is provided with a guide portion 46. The guide portion 46 extends toward a base section of the endoscope (not shown) along the flexible tube 5.

With the extension of the guide portion 46, a lumen 47 is also formed toward the base section of the endoscope along the guide portion 46. In addition, a through-hole 48 which communicates with the lumen 47 is formed in the second forceps piece 22. The other endoscope (not shown) can be inserted into the lumen 47.
The guide portion 46 and the flexible tube 5 are formed so that the guide portion 46 and the flexible tube 5 are insertable into the treatment tool insertion channel (not shown) together. A fill port 46A for filling contrast agent into the guide portion 46 is disposed at the distal end of the guide portion 46.

According to the high-frequency incision forceps 45, in a condition in which the high-frequency incision forceps 45 which is inserted into the treatment tool insertion channel protrudes from the tip of the endoscope, the contrast agent is filled into the lumen 47 through the fill port 46A of the guide portion 46. Thereby, the contrast agent can be applied near the predetermined position to be treated. In addition, similar to the third embodiment, the guide wire 41 is inserted into the lumen 47, and thereby the high-frequency incision forceps 45 can be guided to the tip of the endoscope to move near the predetermined position to be treated. Thus, after filling of the contrast agent, the treatment can be performed using the high-frequency incision forceps 45. As a result, the treatment can be easily performed to reduce the frequency of replacing of the treatment tools.

An other endoscopic treatment tool is inserted into the lumen 47 as another treatment tool insertion channel, and thereby treatments using the first forceps piece 21 and the second forceps piece 22 other than the high-frequency treatment can be performed.

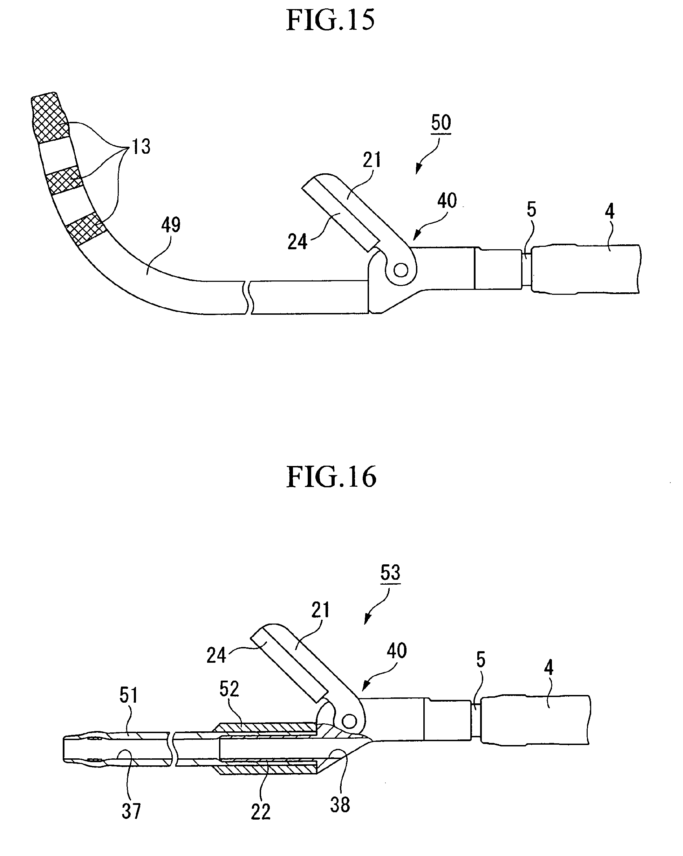

The technical scope of the present invention is not limited to the above embodiments and modifications can be made without departing from the spirit of the present invention. For example, as shown in FIG. 15, in a high-frequency incision forceps 50 of a fifth embodiment of the present invention, the guide portion 49 is formed so as to curve to a predetermined direction by a predetermined curvature. According to the high-frequency incision forceps 50, since the guide portion 49 is inserted into the curved biliary duct 2 easily, the treatment can be performed easily and reliably.

In addition, as shown in FIG. 16, in a high-frequency incision forceps 53 of sixth embodiment of the present invention, an electrical insulation member 52 is disposed around the second forceps piece 22 which is engaged with the guide portion 51. According to the high-frequency incision forceps 53, since the electrode 24 is suppressed to electrically contact the second forceps piece 38 by the insulation member 52 as well as by the guide portion 51, the electrical insulation effects can be improved.

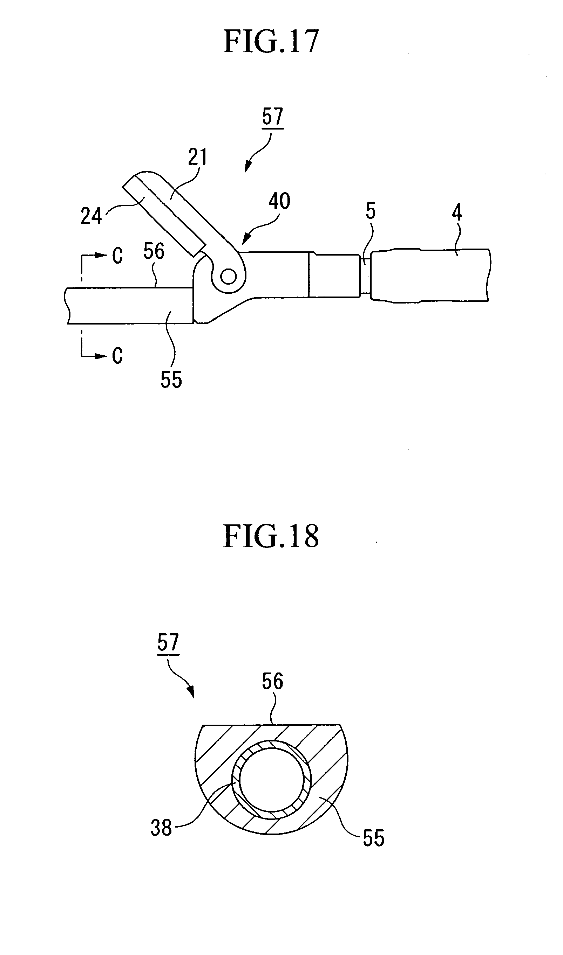

As shown in FIG. 17 and FIG. 18, a high-frequency incision forceps 57 of seventh embodiment of the present invention includes a guide portion 55. A treatment surface 56 of the guide portion 55 is formed so as to be planer. According to the high-frequency incision forceps 57, while a living body tissue (not shown) is grasped between the electrode 24 and the guide portion 55, since the contact area between the living body tissue and the treatment surface 56 is extended, the living body tissue can be grasped reliably.

### INDUSTRIAL APPLICABILITY

The present invention relates to a treatment tool for an endoscope including: an elongate-flexible tube; a first forceps piece and a second forceps piece which are disposed at the tip of the flexible tube and which move so as to open from and close against each other; and a guide portion which is provided at the second forceps piece so as to protrude forward of the tip of the first forceps piece along with extending in a longitudinal direction of the second forceps piece.
According to the treatment tool for an endoscope of the present invention, it is possible to perform the operation easily, and to perform the treatment using the endoscope in the short time.

## Claims

1. A treatment tool for an endoscope, comprising:
an elongate-flexible tube;
a first forceps piece and a second forceps piece which are disposed at a tip of the elongate-flexible tube and which move so as to open and close against each other; and
a guide portion which is provided at the second forceps piece so as to protrude forward of the tip of the first forceps piece along with extending in a longitudinal direction of the second forceps piece.

2. The treatment tool for an endoscope according to Claim 1, wherein
the guide portion is formed so as to be integrated with the second forceps piece.

3. The treatment tool for an endoscope according to Claim 1, wherein
the guide portion is provided with a lumen which is formed in a longitudinal direction of the guide portion and which opens at the tip of the guide portion.

4. The treatment tool for an endoscope according to Claim 3, wherein
the lumen is formed so that a different treatment tool for an endoscope is insertable into the lumen.

5. A treatment tool for an endoscope, comprising:
an elongate-flexible tube;
a first forceps piece and a second forceps piece which are disposed at a tip of the elongate-flexible tube; and
a flexible guide portion which is provided at the second forceps piece so as to protrude forward of the tip of the first forceps piece along with extending in a longitudinal direction of the second forceps piece; wherein
the first forceps piece moves so as to open and close with respect to the second forceps piece.

6. The treatment tool for an endoscope according to Claim 5, wherein
the guide portion is formed so as to be integrated with the second forceps piece.

7. The treatment tool for an endoscope according to Claim 5, wherein
the guide portion is provided with a lumen which is formed in a longitudinal direction of the guide portion and which opens at the tip of the guide portion.

8. The treatment tool for an endoscope according to Claim 7, wherein
the lumen is formed so that a different treatment tool for an endoscope is insertable into the lumen.

9. A treatment tool for an endoscope, comprising:
an elongate-flexible tube which can be inserted into a treatment tool insertion channel provided in an endoscope being insertable into a body cavity and which protrudes from a tip end of the endoscope;
a second forceps piece which is disposed at a tip of the elongate-flexible tube;
a first forceps piece which is disposed at the tip of the flexible tube and which grasps a treated object with the second forceps piece by moving so as to open and close with respect to the second forceps piece; and
a flexible guide portion which is provided at the second forceps piece so as to protrude forward of a tip of the first forceps piece along with extending in a longitudinal direction of the second forceps piece and which extends to a proximal section of the endoscope along the flexible tube.
